# FASCICULE DE BREVET EUROPEEN

(11) **EP 0 959 892 B1**
(45) Date de publication et mention de la délivrance du brevet: **19.02.2003**
(21) Numéro de dépôt: 97913279.2
(22) Date de dépôt: 07.11.1997
(51) Int. Cl.: A61K 31/79, A61P 1/14

(54) **UTILISATION DE PVP OU POVIDONE POUR DIMINUER LE GONFLEMENT INTESTINAL**
VERWENDUNG VON PVP ODER POVIDONE ZUM REDUZIEREN VON DARMSCHWELLUNGEN
USE OF PVP OR POVIDONE FOR REDUCING BOWEL DISTENSION

(30) Priorité: 08.11.1996 FR 9613646; 20.06.1997 FR 9707693; 28.07.1997 FR 9709559; 30.07.1997 FR 9709701
(43) Date de publication de la demande: 01.12.1999
(73) Titulaire: Salkin, André, F-76300 Sotteville les Rouen (FR)
(72) Inventeur: Salkin, André, F-76300 Sotteville les Rouen (FR)
(74) Mandataire: Portal, Gérard
(86) Numéro de dépôt international: FR9702007
(87) Numéro de publication internationale: WO98020883

(56) Documents cités:
- EP-A- 0 273 209
- WO-A-95/17201
- US-A- 5 418 220
- DATABASE WPI Week 9641 Derwent Publications Ltd., London, GB; AN 96-408319 XP002035108 & JP 08 198 761 A (HOKURIKU PHARM.) , 6 août 1996

## Description

L'invention concerne l'utilisation d'au moins un composé chimique organique, pour diminuer ou faire disparaître les variations du tour de taille au cours de la journée.

L'invention concerne aussi une diminution de l'effet constipant de certains composés chimiques.

Les habitudes alimentaires et modes de vie dans les pays industriels favorisent des dérèglements et des déséquilibres physiologiques.

Le présent inventeur a pu observer que ces désordres avaient pour conséquence pour les sujets concernés, et plus particulièrement pour les citadins femmes ou hommes, une variation du tour de taille au cours de la journée, et dont les conséquences sont une sensation de compression dans les vêtements, jupes ou pantalons.

Le présent inventeur a pu observer que certains agents favorables à la diminution du tour de taille produisaient un effet constipant indésirable.

La présente invention a donc pour but de fournir des solutions qui permettent de diminuer ou d'éliminer les variations du tour de taille, tout en supprimant les effets en question.

L'invention fournit une solution nouvelle, techniquement fiable, satisfaisante à l'échelle industrielle, dans le domaine de la production soit de produits de confort, soit de compléments diététiques, soit de produits alimentaires ne favorisant pas les variations du tour de taille.

L'intérêt de cette solution nouvelle réside encore dans le fait qu'elle présente une innocuité complète vis-à-vis de l'organisme.

Ainsi, selon un premier aspect. l'invention conceme l'utilisation d'au moins un agent chimique, d'origine organique, pour diminuer les effets de variation du tour de taille, liés au mode de vie et aux habitudes alimentaires de la population dans les pays industrialisés, en particulier, l'invention concerne l'utilisation d'une polyvinylpyrrolidone ou povidone, en particulier une polyvinylpyrrolidone ou povidone ayant un poids moléculaire compris entre 10 000 et plus de 2,8 millions, soluble ou insoluble, réticulée ou non réticulée, pour diminuer ou éliminer les variations de tour de taille.

Selon un deuxième aspect, l'invention concerne aussi l'utilisation d'une polyvinylpyrrolidone ou povidone, en particulier une polyvinylpyrrolidone ou povidone ayant un poids moléculaire compris entre 10 000 et plus de 2,8 millions. soluble ou insoluble, réticulée ou non réticulée, pour diminuer l'effet de constipation lié à la prise de certains agents tels que le charbon actif, l'argile.

La polyvinylpyrrolidone (PVP) est aussi dénommée povidone dans le Merck Index, 9^{eme} édition, page 996, N°7498. Ces deux termes sont donc équivalents dans le cadre de la présente invention. Le terme polyvinylpyrrolidone regroupe toutes les polyvinylpyrrolidones en particulier les polyvinylpyrrolidones ayant un poids moléculaire compris entre 10000 et plus de 2,8 millions, solubles ou insolubles, réticulées ou non réticulées. Les polyvinylpyrrolidones insolubles sont aussi parfois dénommées polyvinylpolypyrrolidones. De ce fait, l'invention couvre aussi les polyvinylpolypyrrolidones. De telles polyvinylpyrrolidones ou polyvinylpolypyrrolidones sont disponibles, notamment chez GAF®, société américaine, sous les dénominations Plasdone® pour les polyvinylpyrrolidones solubles et Polyplasdone®, pour les polyvinylpyrrolidones ou les polyvinylpolypyrrolidones insolubles.

Selon un autre mode de réalisation avantageux, l'agent est présent sous forme d'une composition distincte à absorber séparément des aliments.

Selon un autre mode de réalisation avantageux, l'agent précité est incorporé directement dans un ou plusieurs aliments.

On peut formuler l'agent sous différentes formes galléniques. De telles formes galléniques et leurs modes de préparation sont familiers à l'homme de l'art.

Selon un autre mode de réalisation avantageux de l'invention, la concentration de l'agent précité est comprise entre une concentration homéopathique et 100 % en poids ou en volume.

Selon un autre mode de réalisation, l'agent en question, c'est-à-dire de préférence la PVP, pourra être formulé en association en synergie avec un autre composé choisi parmi le groupe consistant d'un charbon actif, d'une argile, de préférence une argile montmorillonite, kaolinite, attapulgite, sépiolite, smectite et bentonite. Il s'est révélé que les préparations contenant de la PVP en association avec un ou plusieurs composés actifs cités ci-dessus produisent des effets de synergie tout en diminuant le prix de revient.

La PVP est déjà utilisée dans les compositions alimentaires en tant qu'additif ayant la fonction technologique de raffermissant, stabilisant (SIN : 1201).

Selon le brevet du même déposant enregistré à l'INPI sous le N°9603276 et publié sous la référence FR-A-2 745 981, la PVP peut être utilisée en mélange dans des compositions alimentaires, pour diminuer ou éliminer les effets secondaires liés à l'absorption d'agents édulcorants. Ces effets sont par exemple désordre électrolytique, diarrhée, sensation de torsion digestive.

La PVP a également été utilisée en thérapeutique et a reçu des autorisations de mise sur le marché en tant que médicament notamment dans le cas de maladies entérogastriques. On trouve en particulier le médicament connu en France sous les numéros d'AMM 307 529.2 et 314 449.0.

Dans ce domaine entérogastrique, il existe de nombreux autres médicaments à base d'inhibiteurs de la pompe à protons, de prostaglandines, d'anti-ulcéreux topiques, d'antiacides d'action locale. Ces médicaments peuvent aussi comprendre de l'argile, du charbon actif, de la polyvinylpyrrolidone, des silicones et bien d'autres molécules disponibles. On trouve ces médicaments en France sous les numéros d'AMM 319 230.7 / 319 231.3 / 322 970.8 / 322 971.4 /300 071.0 / 300 072.7 / 330 605.5 / 300 606.1 / 329 851.4 / 330 869.0. Tous ces produits ont pour point commun de traiter des maladies.

Par contre, dans le cadre de la présente invention, on vise a mettre au point un produit de confort. L'objectif est de traiter principatement le bol alimentaire afin que celui ci ne soit pas à l'origine de désordres et de dérèglements augmentant la variation du tour de taille.

Dans le cadre de la présente invention, on vise également à diminuer les effets constipants d'autres agents, tels les argiles, qui ont déjà été utilisées dans le domaine des troubles digestifs.

L'invention couvre aussi une composition, telle que composition alimentaire, boisson, caractérisée en ce qu'elle contient au moins un agent chimique d'origine organique, de préférence la polyvinylpyrrolidone ou povidone en une quantité efficace pour diminuer ou supprimer les variations du tour de taille.

Selon un autre aspect, l'invention concerne une composition telle que composition alimentaire, boisson, caractérisée en ce qu'elle contient au moins un agent chimique d'origine organique, de préférence la polyvinylpyrrolidone ou povidone, en association en synergie avec un autre agent, en particulier argile, en une quantité efficace pour diminuer ou éliminer les variations du tour de taille ou la constipation liée à la prise de l'autre agent.

La présente invention a encore pour objectif des compositions dont le composant actif principal sera au moins un agent chimique d'origine organique précité, de préférence la PVP, seul ou en association en synergie avec d'autres composés actifs tels que charbon, argile, la concentration en agent, de préférence en PVP variant entre une concentration homéopathique et 100% en poids ou en volume, les autres composants pouvant être concentrés de 99,9% à 0%. Ces compositions seront présentées soit sous différentes formes galéniques (gélules, comprimés, cachets, granulés), soit sous forme d'aliments anti-variations du tour de taille tels des boissons, des huiles pour salades, de l'eau potable, du chewing-gum.

L'invention conceme aussi un procédé de traitement du bol alimentaire, caractérisé en ce qu'il comprend l'administration à un être humain s'alimentant avec ledit bol alimentaire, simultanément ou séparément audit bol alimentaire d'une quantité efficace d'au moins un agent chimique, de préférence la polyvinylpyrrolidone ou povidone, formulé seul ou en association en synergie avec d'autres agents tels que le charbon actif, l'argile, de préférence une argile choisie parmi une argile montmorillonite, kaolinite. attapulgite, sépiolite, smectite et bentonite, pour diminuer ou supprimer les variations du tour de taille ou pour diminuer les effets constipants de tels autres agents et en particulier les argiles.

Pour l'un quelconque des aspects de l'invention, au moins un agent chimique d'origine organique précité, avantageusement une polyvinylpyrrolidone ou polyvinylpolypyrrolidoue, peut être utilisé à une concentration depuis une concentration homéopathique jusqu'à 100 % en poids ou en volume selon que la composition est solide ou liquide. Une concentration avantageuse est d'environ au moins 10⁻¹² g à 100 % en poids ou en volume de la composition ou bol alimentaire, à absorber séparément ou de préférence incorporée directement dans un ou plusieurs aliments. Une dose encore préférée sera de l'ordre de 10⁻¹² g à 1 g par dose unitaire, ou pour 100 g de produit à mâcher tel que chewing-gum, ou à consommer.

L'invention peut être formulée sous forme de différentes formes galéniques telles que gélules, comprimés, granulés, en mélange dans des compositions alimentaires telles que compositions buvables ou sucrées, compositions d'huiles alimentaires, eau potable, gommes à mâcher, et les exemples donnés ci-après font partie intégrante de la présente invention, dans les caractéristiques les plus générales.

D'autres buts, caractéristiques et avantages de l'invention apparaîtront clairement à l'homme de fart à partir de la description suivante faite en référence à plusieurs exemples de réalisation donnés simplement à titre d'illustration, et qui ne sauraient donc en aucune façon limiter la portée de l'invention. Dans les exemples, toutes les proportions sont données en poids sauf indication contraire.

### EXEMPLE 1

### PVP formulée sous différentes formes galéniques

La PVP est le seul composant actif, formulé en comprimés, gélules ou granulés.

### a) Cas de gélules

On prépare des gélules de manière classique qui contiennent environ 0,1 g par gélule de polyvinylpyrrolidone, par exemple Plasdone® K29-32 ou Polyplasdone INF 10, disponibles dans le commerce chez GAF®, USA, qui se présentent sous forme de poudre. La composition de la gélule est classique et peut, par exemple, être réalisée en gélatine.

On peut absorber jusqu'à 6 gélules par jour, avant ou après les principaux repas, de manière à fournir de 0.3 à 0.6 g par jour de polyvinylpyrrolidone dont la présence dans le corps permettra de diminuer ou éliminer les variations du tour de taille, causées par les habitudes alimentaires.

### b) Cas de comprimés

Dans ce cas. la polyvinylpyrrolidone, peut être mélangée avec un excipient classique à l'état de poudre, et ensuite comprimée en comprimés. L'excipient que l'on peut utiliser est tout excipient alimentairement ou médicalement acceptable tel que gélatine, stéarate de magnésium. Dans ce cadre la proportion en PVP dans le comprimé sera prévue de manière à fournir une quantité de l'ordre de 0.05 g à 3 g par jour, soit en un comprimé soit en plusieurs comprimés.

### c) Cas de granulés

Les granulés peuvent être formulés pour être avalés facilement avec un peu d'eau Dans ce cas, la PVP peut être ou non associée à un excipient, puis un grain peut être produit par voie sèche ou par voie humide. Dans le cadre d'une posologie, par exemple de 0.5 g par jour, on peut préparer un sachet de 5 g de granulés contenant 0.5 g de PVP.

### EXEMPLE 2

### PVP formulée en mélange dans des compositions alimentaires

### a) Composition buvable sucrée

La PVP selon l'invention peut être incorporée dès la formulation initiale des compositions buvables. Il s'agit ici des compositions buvables sucrées ou contenant des édulcorants autres que le sucre.

Par exemple, dans une boisson à base de coca, on incorporera 1g de Plasdone K29-32 ou K90 disponible dans le commerce chez GAF®, USA, par simple mélange lors de la fabrication.

### b) Composition d'huile alimentaire

Dans ce cas, la PVP est incorporée en mélange après la phase d'extraction. On incorporera par exemple 2 grammes de PVP pour 1 litre d'huile pour salade.

### c) Incorporation dans une eau potable

La PVP peut être ajoutée directement dans une eau potable. On peut mélanger 0,5 g de Polyplasdone INF 10, disponible chez GAF®. USA, à 1 litre d'eau minérale avant son conditionnement en bouteille.

### d) Composition de gomme à mâcher

Dans une composition de gomme à mâcher classique, on ajoutera pour 100 g de la pâte à mâcher 0,5 g de PVP et 0,2 g d'argile montmorillonite.

### ESSAIS COMPARATIFS DEMONTRANT LA DIMINUTION DE VARIATIONS DU TOUR DE TAILLE ET DE CONSTIPATION PAR ABSORPTION DE PVP SELON L'INVENTION APRES DES REPAS.

### EXEMPLE 3

### PVP formulée en préparation avec de l'argile montmorillonite

On réalise selon l'invention des comprimés dosés à 30 mg de PVP et 50 mg de montmorillonite de granulométrie moyenne de l'ordre de 20 µm, et des comprimés dosés à 300 mg de montmorillonite. Des placebos sont réalisés en remplaçant les agents précités par l'agent de compression.

Le test est réalisé sur 21 jours, durant lesquels les sujets consomment deux comprimés après les trois principaux repas, soit 6 comprimés par jour.

On procède à une répartition entre trois groupes, un groupe témoin A consommant un placebo, un groupe B consommant les comprimés de montmorillonite, et un groupe C consommant les comprimés de PVP + montmorillonite.

La mesure du périmètre abdominal est réalisée les jours J1, J3, J5, J8, J10, J12, J15, J17 et J19, pour le repas du midi. Pour chaque jour d'évaluation, la mesure a été effectuée aux heures suivantes : H-1. H0 (heure d'absorption des comprimés), H0.25, H0.5, H1, H1.5.

Les résultats sont exprimés en variation moyenne du périmètre abdominal après les repas par rapport à H-1, en centimètres :
GROUPE A : H0 : +1.5 / H0.25 : + 2,0 / H0,5 : + 1,9 / H1 : +1,9/ H1,5 : +1,3
GROUPE B : H0 : +1,3 / H0,25 : + 1,3 / H0,5 : + 1,0 / H1 : +0,9 / H1,5 : +0,6
GROUPE C : H0 : + 1,2 / H0,25 : + 1.1 / H0,5 : + 1,0 / H1 : + 0.8 / H1.5 + 0,5

L'analyse statistique de ces résultats montre que la différence existant entre le groupe placebo et les deux autres groupes est significative.

La tolérance au traitement était:
GROUPE A: aucune sensation particulière
GROUPE B: problèmes de constipation, surtout la première semaine
GROUPE C: pas de problème particulier

On constate ainsi que l'utilisation de la PVP associée à la montmorillonite selon la présente invention développe une synergie favorisant la diminution de la variation du tour de taille après les repas. De plus, elle résout le problème de constipation rencontré avec l'argile.

### EXEMPLE 4

### Chewing-gum contenant de la PVP

L'essai est réalisé sur deux groupes, un groupe témoin A, consommant des chewing-gums normaux, et un groupe B qui consomme des chewing-gum additionnés de Polyplasdone INF 10 à la concentration de 0,7 g pour 100 g de pâte à mâcher. La prise est de un chewing-gum après chaque repas.

L'essai est réalisé selon la procédure suivante:

### Semaine 1

| | |
|---|---|
| jour 1 | groupe A : sensations de compression après les repas |
| | groupe B : rien à signaler |
| jour 2 | témoin A : gonflement abdominal après les repas |
| | groupe B : sentiment de bien être après les repas |
| jour 3 | témoin A : gonflement abdominal après les repas |
| | groupe B : impression d'une meilleure silhouette du ventre |

On laisse ensuite au repos pendant une semaine (7 jours), puis on inverse la prise, le groupe A prenant le produit auquel a été ajoutée la PVP selon la présente invention, et la groupe B devenant le groupe Témoin B sans PVP. La prise est toujours de trois chewing-gums par jour, un après chaque repas.

### Semaine 2

| | |
|---|---|
| jour 1 | groupe A : rien à signaler |
| | groupe B : sensations de compression après les repas |
| jour 2 | témoin A : sentiment de bien être après les repas |
| | groupe B : gonflement abdominal après les repas |

L'invention permet ainsi, encore une fois, de diminuer les sensations de compression et de gonflement liées aux variations du tour de taille et causées par le mode d'alimentation.

## Revendications

1. Utilisation, à l'exclusion d'une méthode de traitement thérapeutique, d'une polyvinylpyrrolidone pour diminuer ou éliminer les variations de tour de taille.

2. Utilisation, à l'exclusion d'une méthode de traitement thérapeutique, d'une polyvinylpyrrolidone pour diminuer l'effet de constipation de certains composants tels que l'argile.

3. Utilisation selon, la revendication 1 ou 2, **caractérisée en ce qu'**il s'agit d'une utilisation pour éliminer ou diminuer le gonflement abdominal.

4. Utilisation selon l'une des revendications précédentes, **caractérisée en ce que** la polyvinylpyrrolidone a un poids moléculaire compris entre 10 000 et plus de 2,8 millions, et est soluble ou insoluble, réticulée ou non réticulée.

5. Utilisation selon les revendications 1 à 4, **caractérisée en ce que** la polyvinylpyrrolidone est présente sous forme d'une composition distincte à absorber séparément des aliments.

6. Utilisation selon les revendications 1 à 4, **caractérisée en ce que** la polyvinylpyrrolidone est incorporée directement dans un ou plusieurs aliments.

7. Utilisation selon l'une des revendications précédentes, **caractérisée en ce que** la concentration en polyvinylpyrrolidone est comprise entre une concentration homéopathique et 100% en poids ou en volume.

8. Utilisation selon l'une des revendications précédentes, **caractérisée en ce que** la polyvinylpyrrolidone est formulée seule ou en association en synergie avec d'autres agents tels que le charbon actif, l'argile, de préférence une argile montmorillonite, kaolinite, attapulgite, sépiolite, smectite et bentonite.

9. Utilisation selon l'une des revendications précédentes pour fabriquer une composition alimentaire, boisson, **caractérisée en ce qu'**elle contient une polyvinylpyrrolidone à une concentration efficace pour diminuer ou supprimer les variations du tour de taille, en particulier pour diminuer ou éliminer le gonflement abdominal, telle que définie à l'une quelconque des revendications 4 à 8.

10. Utilisation selon l'une des revendications précédentes pour fabriquer une composition alimentaire, boisson, **caractérisée en ce qu'**elle contient une polyvinylpyrrolidone, telle que définie à l'une des revendications 3 à 8, en association en synergie avec un autre agent tel que défini à la revendication 8, en une quantité efficace pour diminuer ou éliminer les variations du tour de taille et la constipation liée à l'autre agent.

11. Utilisation selon l'une des revendications précédentes pour fabriquer une composition selon la revendication 9 ou 10, **caractérisée en ce que** la concentration en polyvinylpyrrolidone est comprise entre une concentration homéopathique et 100 % en poids ou en volume, avantageusement entre environ au moins 10⁻¹² g et 100 % en poids ou en volume, encore mieux entre environ 10⁻¹² g et 1 g par dose unitaire pour 100 g de produits à mâcher ou à absorber.

12. Utilisation selon la revendication 11 dans laquelle le produit à mâcher est un chewing-gum et le produit à absorber est choisi parmi les différentes formes galéniques telles que les gélules, comprimées, granulés, en mélange dans les compositions alimentaires telles que compositions buvables ou sucrées, composition d'huiles alimentaires, eau potable.

13. Utilisation selon la revendication 11 dans laquelle le produit à mâcher est une gomme ou une pâte à mâcher comprenant 0.5 g de polyvinylpyrrolidone et 0.2 g d'argile montmorillonite, pour 100 g de gomme ou de pâte à mâcher.

14. Utilisation selon la revendication 11 dans laquelle le produit à absorber est une gélule comprenant environ 0.1 g de polyvinylpyrrolidone.

15. Utilisation selon la revendication 11 dans laquelle le produit à absorber est un comprimé comprenant de 0.05 à 3 g de polyvinylpyrrolidone.

16. Utilisation selon la revendication 11 dans laquelle le produit à absorber est un granulé comprenant 0.5 g de polyvinylpyrrolidone, pour 5 g de granulés.

17. Utilisation selon la revendication 11 dans laquelle le produit à absorber est une composition buvable sucrée comprenant 1 g de polyvinylpyrrolidone.

18. Utilisation selon la revendication 11 dans laquelle le produit à absorber est une composition d'huile alimentaire comprenant 2 g de polyvinylpyrrolidone par litre d'huile.

19. Utilisation selon la revendication 11 dans laquelle le produit à absorber est une eau potable comprenant 0.5 g de polyvinylpyrrolidone par litre d'eau.

## Claims

1. Use, except in a method of therapeutic treatment, of a polyvinylpyrrolidone for reducing or eliminating changes in waistline.

2. Use, except in a method of therapeutic treatment, of a polyvinylpyrrolidone for reducing the constipating effect of certain components such as clay.

3. Use according to claim 1 or 2, **characterized in that** it is a use for eliminating or reducing abdominal distension.

4. Use according to one of the preceding claims, **characterized in that** the polyvinylpyrrolidone has a molecular weight of between 10,000 and more than 2.8 million, and is soluble or insoluble and crosslinked or non-crosslinked.

5. Use according to claims 1 to 4, **characterized in that** the polyvinylpyrrolidone is present in the form of a distinct composition to be taken separately from foods.

6. Use according to claims 1 to 4, **characterized in that** the polyvinylpyrrolidone is incorporated directly in one or more foods.

7. Use according to one of the preceding claims, **characterized in that** the concentration of polyvinylpyrrolidone is between a homeopathic concentration and 100% by weight or volume.

8. Use according to one of the preceding claims, **characterized in that** the polyvinylpyrrolidone is formulated on its own or in synergistic association with other agents such as activated charcoal or clay, preferably a montmorillonite, kaolinite, attapulgite, sepiolite, smectite or bentonite clay.

9. Use according to one of the preceding claims for the manufacture of a food or drink composition, **characterized in that** it contains a polyvinylpyrrolidone, as defined in any one of claims 4 to 8, at an effective concentration for reducing or eliminating changes in waistline and particularly for reducing or eliminating abdominal distension.

10. Use according to one of the preceding claims for the manufacture of a food or drink composition, **characterized in that** it contains a polyvinylpyrrolidone, as defined in one of claims 3 to 8, in synergistic association with another agent as defined in claim 8, in an effective amount for reducing or eliminating changes in waistline and for reducing or eliminating constipation attributable to the other agent.

11. Use according to one of the preceding claims for the manufacture of a composition according to claim 9 or 10, **characterized in that** the concentration of polyvinylpyrrolidone is between a homeopathic concentration and 100% by weight or volume, advantageously between about at least 10⁻¹² g and 100% by weight or volume and particularly preferably between about 10⁻¹² g and 1 g per unit dose per 100 g of products to be chewed or absorbed.

12. Use according to claim 11 in which the product to be chewed is a chewing gum and the product to be absorbed is selected from various galenical forms such as gelatin capsules, tablets and granules, mixed with food compositions such as drinkable or sweetened compositions, edible oil composition or drinking water.

13. Use according to claim 11 in which the product to be chewed is a chewing gum or jelly comprising 0.5 g of polyvinylpyrrolidone and 0.2 g of montmorillonite clay per 100 g of chewing gum or jelly.

14. Use according to claim 11 in which the product to be absorbed is a gelatin capsule comprising about 0.1 g of polyvinylpyrrolidone.

15. Use according to claim 11 in which the product to be absorbed is a tablet comprising from 0.05 to 3 g of polyvinylpyrrolidone.

16. Use according to claim 11 in which the product to be absorbed consists of granules comprising 0.5 g of polyvinylpyrrolidone per 5 g of granules.

17. Use according to claim 11 in which the product to be absorbed is a sweetened drinkable composition comprising 1 g of polyvinylpyrrolidone.

18. Use according to claim 11 in which the product to be absorbed is an edible oil composition comprising 2 g of polyvinylpyrrolidone per litre of oil.

19. Use according to claim 11 in which the product to be absorbed is a drinking water comprising 0.5 g of polyvinylpyrrolidone per litre of water.

## Patentansprüche

1. Verwendung, unter Ausschluss eines therapeutischen Behandlungsverfahrens, eines Polyvinylpyrrolidons zur Verminderung oder Beseitigung von Variationen beim Körperumfang.

2. Verwendung, unter Ausschluss eines therapeutischen Behandlungsverfahrens, eines Polyvinylpyrrolidons zur Verminderung der Verstopfungswirkung bestimmter Bestandteile wie von Ton.

3. Verwendung gemäß Anspruch 1 oder 2, **dadurch gekennzeichnet, dass** es sich um eine Verwendung zur Beseitigung oder Verminderung von Magenblähungen handelt.

4. Verwendung gemäß einem der vorhergehenden Ansprüche, **dadurch gekennzeichnet, dass** das Polyvinylpyrrolidon ein Molekulargewicht von 10000 bis mehr als 2800000 aufweist und löslich oder unlöslich, vernetzt oder unvernetzt ist.

5. Verwendung gemäß einem der Ansprüche 1 bis 4, **dadurch gekennzeichnet, dass** das Polyvinylpyrrolidon in Form einer bestimmten Zusammensetzung vorliegt, um Nahrungsmittel getrennt zu absorbieren.

6. Verwendung gemäß einem der Ansprüche 1 bis 4, **dadurch gekennzeichnet, dass** das Polyvinylpyrrolidon direkt in ein oder mehrere Nahrungsmittel eingebracht wird.

7. Verwendung gemäß einem der vorhergehenden Ansprüche, **dadurch gekennzeichnet, dass** die Konzentration an Polyvinylpyrrolidon im Bereich von einer homöopathischen Konzentration bis 100 Gew.% oder Volumen% liegt.

8. Verwendung gemäß einem der vorhergehenden Ansprüche, **dadurch gekennzeichnet, dass** das Polyvinylpyrrolidon alleine oder in Synergie-Zusammensetzung mit weiteren Mitteln wie Aktivkohle und Ton, vorzugsweise einem Ton aus Montmorillonit, Kaolinit, Attapulgit, Sepiolit, Smektit und aus Bentonit, formuliert wird.

9. Verwendung gemäß einem der vorhergehenden Ansprüche zur Herstellung einer Nahrungsmittel-Getränkzusammensetzung, **dadurch gekennzeichnet, dass** diese ein in einem der Ansprüche 4 bis 8 definiertes Polyvinylpyrrolidon in einer Konzentration enthält, die wirksam ist, um die Variationen des Körperumfangs, insbesondere Magenblähungen zu vermindern oder zu unterdrücken.

10. Verwendung gemäß einem der vorhergehenden Ansprüche zur Herstellung einer Nahrungsmittelgetränkzusammensetzung, **dadurch gekennzeichnet, dass** diese ein in einem der Ansprüche 3 bis 8 definiertes Polyvinylpyrrolidon in Synergie-Zusammensetzung mit einem in Anspruch 8 definierten weiteren Mittel in einer Menge enthält, die wirksam ist, um die Variationen des Körperumfangs und die mit dem weiteren Mittel zusammenhängenden Verstopfungen zu vermindern oder zu beseitigen.

11. Verwendung gemäß einem der vorhergehenden Ansprüche zur Herstellung einer in Anspruch 9 oder 10 definierten Zusammensetzung, **dadurch gekennzeichnet, dass** die Konzentration von Polyvinylpyrrolidon im Bereich einer homöopathischen Konzentration bis 100 Gew.% oder Volumen% und in vorteilhafter Weise von ca. mindestens 10⁻¹² g bis 100 Gew.% oder Volumen% und noch besser von ca. 10⁻¹² g bis 1 g pro Einheitsdosis für 100 g Produkte zum Kauen oder Absorbieren liegt.

12. Verwendung gemäß Anspruch 11, worin das Produkt zum Kauen ein Kaugummi ist und das Produkt zum Absorbieren aus unterschiedlichen galenischen Formen wie aus Gelatinekapseln, Tabletten und aus Körnern in Mischung in Nahrungsmittelzusammensetzungen wie trinkbaren oder gezuckerten Zusammensetzungen, einer Zusammensetzung aus Nahrungsmittelölen und trinkbarem Wasser ausgewählt ist.

13. Verwendung gemäß Anspruch 11, worin das Produkt zum Kauen ein Gummi oder eine Paste zum Kauen ist, umfasend 0,5 g Polyvinylpyrrolidon und 0,2 g Montmorillonit-Ton für 100 g Gummi oder Paste zum Kauen.

14. Verwendung gemäß Anspruch 11, worin das Produkt zum Absorbieren eine Gelatinekapsel ist, umfassend ca. 0,1 g Polyvinylpyrrolidon.

15. Verwendung gemäß Anspruch 11, worin das Produkt zum Absorbieren eine Tablette ist, umfassend 0,05 bis 3 g Polyvinylpyrrolidon.

16. Verwendung gemäß Anspruch 11, worin das Produkt zum Absorbieren ein Korn ist, umfassend 0,5 g Polyvinylpyrrolidon für 5 g Körner.

17. Verwendung gemäß Anspruch 11, worin das Produkt zum Absorbieren eine trinkbare gezuckerte Zusammensetzung ist, umfassend 1 g Polyvinylpyrrolidon.

18. Verwendung gemäß Anspruch 11, worin das Produkt zum Absorbieren eine Zusammensetzung aus Nahrungsmittelöl ist, umfassend 2 g Polyvinylpyrrolidon pro Liter Öl.

19. Verwendung gemäß Anspruch 11, worin das Produkt zum Absorbieren ein trinkbares Wasser ist, umfassend 0,5 g Polyvinylpyrrolidon pro Liter Wasser.
